# EUROPÄISCHE PATENTSCHRIFT

(11) **EP 1 817 399 B1**
(45) Veröffentlichungstag und Bekanntmachung des Hinweises auf die Patenterteilung: **11.08.2010**
(21) Anmeldenummer: 05803485.1
(22) Anmeldetag: 03.11.2005
(51) Int. Cl.: C11D 3/395, C11D 3/39, C11D 17/00, A61L 9/05

(54) **HAFTENDES SANITÄRREINIGUNGS- UND BEDUFTUNGSMITTEL**
ADHESIVE AGENT FOR SANITARY CLEANING AND DEODORIZATION
AGENT ADHESIF DE NETTOYAGE ET DE DESODORISATION SANITAIRE

(30) Priorität: 23.11.2004 DE 102004056554
(43) Veröffentlichungstag der Anmeldung: 15.08.2007
(73) Patentinhaber: Buck-Chemie GmbH, 71083 Herrenberg (DE)
(72) Erfinder: DETTINGER, Johannes, 72160 Horb (DE); FRITZ, Matthias, 72810 Gomaringen (DE); JAESCHKE, Edgar, 70794 Filderstadt (DE)
(74) Vertreter: Mammel, Ulrike
(86) Internationale Anmeldenummer: PCT/EP2005/011770
(87) Internationale Veröffentlichungsnummer: WO 2006/056301

(56) Entgegenhaltungen:
- EP-A- 0 864 637
- WO-A-02/12431
- DE-A1- 10 047 298
- DE-A1- 19 715 872
- US-A- 5 827 810
- US-A- 6 100 228

## Beschreibung

Die Erfindung betrifft ein haftendes Sanitärmittel zum Reinigen und/oder Desinfizieren und/oder zur Duftstoffabgabe für Sanitärgegenstände wie Toilettenspülbecken.

Diese Sanitärmittel sind viskose, im allgemeinen pastöse Gele, die aus einem entsprechenden Behältnis direkt auf der Oberfläche des Sanitärgegenstandes aufgebracht werden, dort haften und erst nach einer größeren Anzahl von Spülvorgängen abspülbar sind. Im Allgemeinen sind diese Mittel wenigstens teilweise flüssigkristallin.

Durch die direkte Haftung des Mittels auf der Oberfläche des Sanitärgegenstandes ist es nicht erforderlich, zusätzlich Behältnisse wie die sogenannten "WC-Körbchen" vorzusehen, deren Benutzung vom Verbraucher insbesondere beim Ersetzen des Sanitärmittels und beim Putzen der Toilette als unhygienisch empfunden wird.

Solche haftenden Sanitärmittel sind aus der WO 99/66017 bekannt. Sie umfassen neben Tensiden, Wasser und Duftstöffen auch Haftvermittler, beispielsweise aus der Klasse der nichtionischen Tenside wie den Polyalkoxyalkanen und sind viskose, feste oder pastöse Mittel. Eine polarisationsmikroskopische Untersuchung der aus dieser Druckschrift bekannten Reinigungsmittel zeigt, dass die Mittel flüssigkristalline Phasen, insbesondere aus hexagonalen Strukturen, umfassen.

Um ein Austrocknen der Oberfläche dieser haftenden Mittel zu verhindern und eine unabhängig von der Anzahl der Spülungen ansehnliche und glatte Oberfläche bereitzustellen, wird in der DE 100 48 887 A1 vorgeschlagen, dem Mittel zusätzlich eine aliphatische Di-, Oligo- oder Polyhydroxyverbindung oder deren Ether zuzusetzen.

Aus der EP 1 318 191 ist ein Sanitärmittel bekannt, das als Haftvermittler Verbindungen aus der Gruppe der Oligo- oder Poyethylenoxid und/oder Oligo- und/oder Polypropylenoxid und/oder Oligo- und/oder Polybutylenoxid umfassenden Blockpolymere, der Arylethoxylate oder der Alkyl-Arylethoxylate umfasst.

Diese bekannten haftenden Sanitärmittel lassen sich auf einfache und hygienische Art und Weise mit einer geeigneten Vorrichtung applizieren, haften an der Oberfläche des Sanitärgegenstands, behalten ihre Form bei und werden selbst unter Einwirkung von Wasser nicht als Ganzes herabgespült, sondern lösen sich erst nach einer Vielzahl von Spülungen nach und nach vollständig auf.

Weiterhin sind aus der DE 103 56 254 A1 lang haftende Sanitärmütel bekannt geworden, die anionische Tenside und nichtionische Tenside als Haftvermittler und Silikate als Verdicker umfassen.

Diese Mittel zeichnen sich durch gute Handhabbarkeit und eine gute Reinigungswirkung aus. Durch die direkte Applizierbarkeit des Mittels ist es nicht mehr erforderlich, dass der Verbraucher beim Nachfüllen ein möglicherweise verunreinigtes Toilettenkörbchen berühren muss. Auch kann der Verbraucher das Mittel in der von ihm gewünschten Menge aufbringen und ist nicht auf die von Hersteller vorgesehene Packungsmenge beschränkt.

Ein weiterer Vorteil der bekannten gelförmigen Mittel ist, dass sich diese durch eine im Vergleich zu den Reinigungsmittelformkörpern deutlich bessere Duftfreisetzung auszeichnen.

Die WO 02/12431 lehrt pastenförmige Reinigungs- und Desinfektionsmittel, die als Bleichmittel für Wäsche, zur Oberflächendesinfektion in Krankenhäusern und in Küchen und von Fußböden verwendet werden.

Aus der EP-A-0864637 sind Sanitärmittel zur Reinigung und Beduftung bekannt. Diese Mittel sind stückförmig und werden in einem käfigartigen Behälter oder Korb im Toilettenbecken an einer Stelle befestigt, die bei jedem Spülvorgang von zulaufendem Spülwasser durchströmt wird.

Reinigungs- und Beduftungsflüssigkeiten für Toiletten zur Anwendung in einem Flüssigdosiergerät sind aus der DE 100 47 298 A1 bekannt.

Zur Verbesserung der Reinigungs- und Desinfektionskraft ist bei extrudierten Reinigungsmittelformkörpern wie den Rimblocks oder auch den In-Tank-Blocks bekannt, halogenfreisetzende Mittel als Bleichmittel zu verwenden. Allerdings treten bei diesen bekannten, halogenfreisetzende Substanzen umfassenden.Mitteln Schwierigkeiten in Bezug auf die Stabilität auf, wie beispielsweise in der EP 0 462 643 A1, der WO 92/18605 und der EP 0 672 103 B1 beschrieben. Gerade unter feuchten Bedingungen wurde in den bekannten, halogenfreisetzende Mittel umfassenden Blocks eine Zersetzung der halogenfreisetzenden Mittel und eine unerwünschte Reaktion der halogenfreisetzenden Mittel mit anderen reaktiven Bestandteilen beobachtet, und es wurden in den obigen Druckschriften und einer Vielzahl weiterer Publikationen eine Vielzahl von Vorschlägen unterbreitet, wie das Problem der Zersetzung der Bleichmittel und des Angriffs der Bleichmittel auf andere empfindliche Substanzen in den bekannten Reinigungsmitteln gelöst werden kann. Nicht zuletzt wurde wegen dieser Schwierigkeiten gar vorgeschlagen, die Reinigungsmittel mehrphasig vorzusehen, nämlich mit einer Bleichmittel umfassenden Phase und einer bleichmittelfreien Phase mit den empfindlichen Substanzen.

Die Aufgabe der vorliegenden Erfindung besteht darin, ein Toilettenreinigungs- und/oder Beduftungsmittel bereitzustellen, das bei einfacher und hygienischer Handhabbarkeit eine hohe Reinigungs- und Desinfektionskraft aufweist.

Erstaunlicherweise wurde festgestellt, dass es trotz der aus dem Stand der Technik bekannten Instabilität von Bleichmitteln gerade in feuchter Umgebung möglich ist, ein unmittelbar auf dem Sanitärgegenstand applizierbares Mittel, das Wasser, Haftvermittler und anionische Tenside umfasst, mit sauerstofffreisetzenden Bleichmitteln bereitzustellen. Erstaunlicherweise tritt die aus dem Stand der Technik bekannte hohe Instabilität der sauerstofffreisetzenden Substanzen in den gelförmigen, die Haftvermittler umfassenden Reinigungsmitteln nicht oder wenigstens nur in einem geringen Maße auf.

Nach Ansicht der Anmelderin könnte dies möglicherweise darauf zurückzuführen sein, dass eine durch rauhe Oberflächen katalysierte Zersetzung der sauerstofffreisetzenden Substanzen in dem Mittel gerade durch die Gelstruktur nicht stattfindet.

Die erfindungsgemäßen Mittel sind flüssigkristalline Gele. Vorzugsweise weisen die Gele eine hexagonale Struktur auf.

Bei den erfindungsgemäßen sauerstofffreisetzenden Bleichmitteln handelt es sich um die Verbindungen aus der Gruppe der Carbonat Peroxyhydrate, Perborate, Persulfate, Peroxodisulfate, Peroxophosphate, Peroxonitrate, Caroate, Nitrate, Dithionite, Chlorate, Permanganate jeweils in der Form ihrer Alkali-, Ammonium- oder Erdalkalisalze.

Ferner handelt es sich um die Wasserstoffperoxide und organischen Peroxide.

Unter den Alkalisalzen sind die Natrium- und Kaliumsalze und unter den Erdalkalisalzen Calcium besonders bevorzugt.

Besonders bevorzugt ist Wasserstoffperoxid als Bleichmittel zu verwenden.

Die erfindungsgemäßen sauerstofffreisetzenden Mittel können in dem Gel in einem Anteil von 1 bis zu 20 Gew.% enthalten sein, vorzugsweise beträgt ihr Anteil zwischen 2 und 15 Gew.% und besonders bevorzugt zwischen 5 und 10 Gew.%.

Im Allgemeinen sind diese Verbindungen im Wesentlichen im Gel stabil. Sofern gewünscht, kann jedoch eine zusätzliche Stabilisierung des Bleichmittels durch den Zusatz eines Stabilisierungsmittels erfolgen. Als Stabilisierungsmittel können Harnstoff, Barbitursäure, hydrophobe Verbindungen wie beispielsweise Mineralöle oder sonstige unreaktive hydrophobe Verbindungen wie tertiäte Alkohole, komplexe Ester oder Ketone, eingesetzt werden. U.U. können diese hydrophoben Stabilisierungsmittel auch gleichzeitig Parfümierungsstoffe und/oder Löslichkeitskontrollmittel sein. Als Stabilisierungsmittel können auch Komplexbildner wie Phosphorsäure, Natriumdiphosphate, Stannate, Silikate wie Erdalkalisilikate, EDTA, NTA und Citrate ausgewählt werden, beispielsweise bei den bevorzugt sauerstofffreisetzenden Bleichmitteln Wasserstoffperoxide, die eine Zersetzung katalysierenden Schwermetalle komplexieren.

Besonders bevorzugt ist, als erfindungsgemäßes Bleichmittel Wasserstoffperoxid einzusetzen. Wasserstoffperoxid wird vorzugsweise in einer Konzentration von 30 % in Wasser, ggf. stabilisiert, eingesetzt. Wasserstoffperoxid lässt sich als Flüssigkeit bzw. wässrige Lösung direkt zur Herstellung des Mittels einsetzen, ohne dass beispielsweise das Auflösen eines Salzes oder ähnliches erforderlich wird. Des Weiteren ist Wasserstoffperoxid in dem Gel erstaunlich stabil. Die Oxidationswirkung des Wasserstoffperoxids ist zudem selektiver als bei den entsprechenden chlorabspaltenden Substanzen.

Ein weitere Vorteil des Wasserstoffperoxids ist, dass weder das Wasserstoffperoxid, noch seine Oxidationsprodukte, nämlich Sauerstoff, farbig sind und somit das Aussehen des Mittels beeinträchtigen können, wie das bei einem Einsatz von Bleichmitteln auf Brom - oder Jodbasis, die u. U. anschließend zu einer Braunfärbung des Mittels führen können, der Fall wäre.

In Abhängigkeit von der gewünschten Farbe und auch Eigenfarbe können jedoch durchaus auch Jod oder Brom freisetzende Mittel eingesetzt werden, insbesondere, wenn das Gel empfindlichere Bestandteile aufweist, da das Oxidationspotential der Brom und Jod freisetzenden Mittel geringer als das der Chlor oder Sauerstoff freisetzenden Mittel ist.

Um eine katalytische Zersetzung des Wasserstoffperoxids in dem Gel zu verhindern, sollte das Gel frei von Schwermetallen wie bspw. Braunstein, Fe³⁺, Ca²⁺ sowie frei von J⁻, JO₃⁻ sein.

Sofern die Oxidationskraft des Bleichmittels in dem Gel gesteigert werden soll, beispielsweise neue reaktionsträgere Bleichmittel wie Perborate eingesetzt werden, können von Bleichaktivatoren, insbesondere auf der Basis von N- oder O-Acylverbindungen, wie zum Beispiel Tetraacetylethylendiamin (TAED), Tetraacetylglycoluril (TAGU), Pentapropionylglucose (PPG) oder Pentaacetylglycose (PAG) zugesetzt werden.

Der Haftvermittler bewirkt in Anwesenheit von Wasser, daß das Mittel an der Oberfläche haftet. Im allgemeinen bildet er netzwerkartige Strukturen aus, die dem Mittel selbst unter der starken Krafteinwirkung durch Spülwasser die erforderliche Formbeständigkeit verleihen.

Die Moleküle des Haftvermittlers sind länger- oder langkettige, im wesentlichen gestreckte Moleküle, die wenigstens teilweise hydrophil sind und somit wenigstens einen hydrophilen Rest oder eine hydrophile Gruppe umfassen, die mit Wasser wechselwirkt.

Vorzugsweise sollte es sich bei den Haftvermittlern um unverzweigte Moleküle handeln, um die gewünschte Netzwerkbildung zu ermöglichen.

Der Haftvermittler kann entweder insgesamt hydrophil sein oder auch teilweise hydrophil, teilweise hydrophob.

Als Haftvermittler können organische Moleküle mit einem hydrophilen und einem hydrophoben Ende eingesetzt werden. Als hydrophile Reste können beispielsweise Polyalkoxygruppen, vorzugsweise Polyethoxy-, Polypropoxy- oder Polybutoxy- oder auch gemischte Polyalkoxygruppen wie beispielsweise Poly(ethoxypropoxy)gruppen eingesetzt werden. Besonders bevorzugt ist, als hydrophiles Ende einen Polyethoxyrest; umfassend zwischen 15 und 100 Ethoxygruppen, vorzugsweise zwischen 25 und 55 und besonders bevorzugt 35 +/- 5 Ethoxygruppen, zu verwenden.

Als hydrophiles Ende können auch anionische Gruppen, beispielsweise Sulfonate, Carbonate oder Sulfate eingesetzt werden.

Auch Stearate, insbesondere Natrium oder Kaliumstearat, sind als Haftvermittler geeignet.

Sofern die Haftvermittlermoleküle auch ein hydrophobes Ende aufweisen, sind für den hydrophoben Rest insbesondere geradkettige Alkylreste geeignet, wobei insbesondere geradzahlige Alkylreste wegen der besseren biologischen Abbaubarkeit besonders bevorzugt sind. Um die gewünschte Netzwerkbildung der Haftvermittlermoleküle zu erreichen, sollten die Moleküle unverzweigt sein.

Werden als hydrophobe Reste Alkylgruppen ausgewählt, so sind Alkylreste mit wenigstens zwölf Kohlenstoffatomen bevorzugt. Besonders gute Ergebnisse wurden mit einer Alkylkettenlänge zwischen 16 und 30 Kohlenstoffatomen, insbesondere 16, 18, 20 und 22 Kohlenstoffatomen, erzielt.

Als hydrophobe Reste können die Haftvermittler auch Alkylbenzolreste umfassen, beispielsweise Dodecylbenzol oder ähnliche Reste, wie es bei dem Haftvermittler Natriumdodecylbenzolsulfonat der Fall ist.

Eine große Klasse der Haftvermittler bilden die nichtionischen Tenside und die nichtionischen Emulgatoren.

Als nichtionische Tenside werden erfindungsgemäß vorzugsweise alkoxylierte, vorteilhafterweise ethoxylierte und/oder propoxylierte, besonders bevorzugt ethoylierte, primäre lineare Alkohole, insbesondere Fettalkohole, und/oder Fettsäuren mit vorzugsweise 8 bis 22, besonders bevorzugt 12 bis 22, C-Atomen und durchschnittlich 5 bis 120 Mol Ethylenoxid (EO) und/oder 1 bis 5 Mol Propylenoxid (PO) pro Mol Alkohol eingesetzt, in denen der Alkoholrest linear oder, bevorzugt in 2-Stellung, methylverzweigt sein kann beziehungsweise lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 22 C-Atomen, zum Beispiel aus Kokos-, Palm-, Talgfett- oder Oleylalkohol, und durchschnittlich 20 bis 100, insbesondere 20 bis 80 EO pro Mol Alkohol bevorzugt. Zu den bevorzugten ethoxylierten Alkoholen gehören beispielsweise C₁₂₋₁₄-Alkohole und/oder Talgfettalkohole mit 30, 40, 50 oder 60 EO-Einheiten und Mischungen aus diesen. Die angegebenen Ethoxylierungsgrade stellen gegebenenfalls statistische Mittelwerte dar, die für ein spezielles Produkt eine ganze oder eine gebrochene Zahl sein können. Bevorzugte Alkoholethoxylate weisen eine eingeengte Homologenvertellung auf (narrow range ethoxylates, NRE).

Bevorzugte Haftvermittler sind Polyalkoxyalkane, vorzugsweise ein Gemisch aus Alkyl(C20-C20)-ethoxylat mit 35 EO beziehungsweise Alkyl(C22)-ethoxylat mit 35 EO und Alkyl(C16-C18)-ethoxylat mit 30 EO, aber auch Alkylsulfonate wie Natriumdodecylbenzolsulfonat oder Alkylcarbonate oder Alkylsulfate.

Die Verwendung eines Gemischs der Haftvermittler Alkyl(C22)-ethoxylat (35EO) und Alkyl(C16-C18)-ethoxylat (30EO) wirkt sich auf die gewünschte Spülzahl im Sinne einer Reduktion und die Standfestigkeit des Mittels positiv aus. Darüber hinaus wird durch die gemeinsame Verwendung dieser Haftvermittler eine einfache Verarbeitbarkeit des Mittels erreicht.

Mit Verringerung der Anzahl der Alkoxygruppen wird der Haftvermittler lipophiler, wodurch beispielsweise die Löslichkeit von Parfum und damit die Intensität der Beduftung erhöht werden kann.

Weiterhin bevorzugt sind erfindungsgemäß C₁₂-C₂₂-Fettsäuremono- und -diester von Anlagerungsprodukten von 10 bis 150 Mol Ethylenoxid an Polyole mit 3 bis 6 Kohlenstoffatomen, insbesondere an Glycerin, C₈-C₂₂-Alkylmono- und -oligoglycoside und deren ethoxylierte Analoga, Anlagerungsprodukte von 20 bis 150, insbesondere 20 bis 100, vor allem 20 bis 80 Mol Ethylenoxid an Rizinusöl oder gehärtetes Rizinusöl, Partialester von Polyolen mit 3 - 6 Kohlenstoffatomen mit gesättigten Fettsäuren mit 8 bis 22 C-Atomen, Fettsäureester von Zuckern und Zuckeralkoholen wie Sorbit, die gegebenenfalls mit 4 bis 150 Mol Ethylenoxid und/oder 0 bis 5 Mol Propylenoxid alkoxyliert sein können. Gehärtetes Rizinusöl, das mit 40 EO-Einheiten alkoxyliert wurde, ist beispielsweise unter dem Handelsnamen Eumulgin^{®} HRE 40 (Cognis) erhältlich. Rizinusöl, das mit 60 EO-Einheiten alkoxyliert wurde, ist beispielsweise unter den Handelsnamen Eumulgin^{®} HRE 60 (Cognis) und Cremophor^{®} CO60 (BASF) erhältlich.

Eine weitere Klasse erfindungsgemäß einsetzbarer nichtionischer Tenside, die entweder als alleiniges nichtionisches Tensid oder in Kombination mit anderen nichtionischen Tensiden eingesetzt werden können, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die erfindungsgemäß eingesetzt werden können, sind die Alkylpolyglycoside (APG). Einsetzbare Alkypolyglycoside haben die allgemeine Formel R-O(-G)₂, in der R für einen linearen oder verzweigten, insbesondere in 2-Stellung methylverzweigten, gesättigten oder ungesättigten, aliphatischen Rest mit 8 bis 22, vorzugsweise 8 bis 18 C-Atomen, steht, G für einen glykosidisch gebundenen Rest eines Monosaccarids steht und z ein Wert zwischen 1 und 10 bedeutet.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette.

Auch nichtionische Tenside vom Typ der Aminoxide, beispielsweise N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxlierten Fettalkohole, insbesondere nicht mehr als die Hälfte davon.

Weitere geeignete Tenside sind Polyhydroxyfettsäureamide der Formel (II), 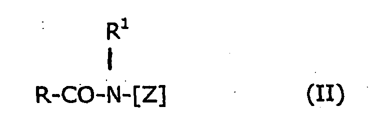 in der RCO für einen aliphatischen Acylrest mit 6 bis 22 Kohlenstoffatomen, R¹ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel (III), 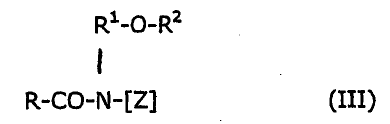 in der R für einen linearen oder verzweigten Alkyl- oder Alkenylrest mit 7 bis 12 Kohlenstoffatomen, R¹ für einen linearen, verzweigten oder cyclischen Alkylrest oder einen Arylrest mit 2 bis 8 Kohlenstoffatomen und R² für einen linearen, verzweigten oder cyclischen Alkylrest oder einen Oxy-Alkylrest mit 1 bis 8 Kohlenstoffatomen steht, wobei C₁₋₄-Alkyl- oder Phenylreste bevorzugt sind und [Z] für einen linearen Polyhydroxyalkylrest steht, dessen Alkylkette mit mindestens zwei Hydroxylgruppen substituiert ist, oder alkoxylierte, vorzugsweise ethoxylierte oder propxylierte Derivate dieses Restes.

Die nichtionischen Tenside sind vorzugsweise in einer Konzentration von 10 - 60, besonders bevorzugt 10 - 30, vor allem 15 - 25 Gew.%, im Gel enthalten.

Es können auch Moleküle als Haftvermittler eingesetzt werden, die in wässrigen Systemen allgemein als Verdicker wirken, zum Beispiel hydrophile Substanzen wie Cellulosen, Polysaccharide, Stärke, Xanthan-Gum, Agar-Agar, Gellan-Gum, Gummi-Arabicum, Johannisbrotkernmehl oder Guar-Gum.

Die einzusetzende Konzentration des Haftvermittlers ist von dessen Hydrophilie und dessen Netzwerkbildungsvermögen abhängig. Sie können beispielsweise 10 und 40 Gew.%, vorzugsweise zwischen 15 und 35 Gew.% und besonders bevorzugt zwischen 20 und 30 Gew.%, betragen, was insbesondere bei der Verwendung Polyalkoxyalkanen bevorzugt ist.

Um mit den Haftvermittlermolekülen durch Anlagerung von Wasser die gewünschte Anzahl von Klebestellen bereitzustellen, sollte Wasser wenigstens zu 20 Gew.%, vorzugsweise zwischen 35 und 65 Gew.%, insbesondere zwischen 40 und 60 Gew.%, in der Formulierung enthalten sein. Der Wasseranteil ist unter anderem von der Hydrophilie des eingesetzten Haftvermittlers abhängig.

Zusätzlich können als Lösungsmittel, insbesondere für Farbstoffe und Parfümöle, beispielsweise Alkanolamine, Polyole wie Ethylenglycol, Propylenglycol, 1,2 Glycerin und andere ein- und mehrwertige Alkohole enthalten sein. Der Gehalt der Lösungsmittel ist abhängig von der Art und Menge der zu lösenden Bestandteile und liegt in der Regel im Bereich von 0,5 bis 5 Gew%.

Als Tenside können prinzipiell alle bekannten anionischen und kationischen oder amphoteren Tenside eingesetzt werden.

Als anionische Tenside werden beispielsweise solche vom Typ der Sulfonate und Sulfate eingesetzt. Als Tenside vom Sulfonat-Typ kommen dabei vorzugsweise C₉₋₁₃-Alkylbenzolsulfonate, Olefinsulfonate, das heißt Gemische aus Alkan- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält, in Betracht.

Geeignet sind auch Alkansulfonate, die aus C₁₂₋₁₈-Alkanen, beispielsweise durch Sulfochlorierung oder Sulfoxidation mit anschließender Hydrolyse beziehungsweise Neutralisation gewonnen werden. Ebenso sind auch die Ester von α-Sulfofettsäuren (Estersulfonate), zum Beispiel die α-sulfonierten Methylester der hydrierten Kokos-, Palmkern- oder Talgfettsäuren geeignet.

Weitere geeignete anionische Tenside sind sulfierte Fettsäureglycerinester. Unter Fettsäureglycerinestern sind die Mono-, Di- und Triester sowie deren Gemische zu verstehen, wie sie bei der Herstellung durch Veresterung von einem Monogylcerin mit 1 bis 3 Mol Fettsäure oder bei der Umesterung von Triglyceriden mit 0,3 bis 2 Mol Glycerin erhalten werden. Bevorzugte sulfierte Fettsäureglycerinester sind dabei die Sulfierprodukte von gesättigten Fettsäuren mit 6 bis 22 Kohlenstoffatomen, beispielsweise der Capronsäure, Caprylsäure, Caprinsäure, Myristinsäure, Laurinsäure, Palmitinsäure, Steanrinsäure oder Behensäure.

Als Alk(en)ylsulfate werden die Alkali- und insbesondere die Natriumsalze der Schwefelsäurehalbester der C₁₂-C₁₈-Fettalkohole, beispielsweise aus Kokosfettalkohol, Talgfettalkohol, Lauryl-, Myristyl-, Cetyl- oder Stearylalkohol oder der C₁₀-C₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂- C₁₈-Alkylsulfate und C₁₂-C₁₅-Alkylsulfate sowie C₁₄-C₁₅-Alkylsulfate bevorzugt. Auch 2,3-Alkylsulfate, welche als Handelsprodukte der Shell Oil Company unter dem Namen DAN^{®} erhalten werden können, sind geeignete Aniontenside.

Auch die Schwefelsäuremonoester der mit 1 bis 6 Mol Ethylenoxid ethoxylierten geradkettigen oder verzweigten C₇₋₂₁-Alkohole, wie 2-Methylverzweigte C₉₋₁₁-Alkohole mit im Durchschnitt 3,5 Mol Ethylenoxid (EO) oder C₁₂₋₁₈-Fettalkohole mit 1 bis 4 EO, sind geeignet. Sie werden hierbei aufgrund ihres hohen Schaumverhaltens nur in relativ geringen Mengen eingesetzt.

Weitere geeignete anionische Tenside sind auch die Salze der Alkylsulfobernsteinsäure, die auch als Sulfosuccinate oder als Sulfobernsteinsäureester bezeichnet werden und die Monoester und/oder Diester der Sulfobernsteinsäure mit Alkoholen, vorzugsweise Fettalkoholen und insbesondere ethoxilierten Fettalkoholen darstellen. Bevorzugte Sulfosuccinate enthalten C₈₋₁₈-Fettalkoholreste oder Mischungen aus diesen. Insbesondere bevorzugte Sulfosuccinate enthalten einen Fettalkoholrest, der sich von ethoxylierten Fettalkoholen ableitet, die für sich betrachtet nichtionische Tenside darstellen (Beschreibung siehe unten). Dabei sind wiederum Sulfosuccinate, deren Fettalkohol-Reste sich von ethoxylierten Fettalkoholen mit eingeengter Homologenverteilung ableiten, besonders bevorzugt. Ebenso ist es auch möglich, Alk(en)ylbernsteinsäure mit vorzugsweise 8 bis 18 Kohlenstoffatomen in der Alk(en)ylkette oder deren Salze einzusetzen. Die anionischen Tenside können in Form ihrer Natrium-, Kalium- oder Ammoniumsalze sowie als lösliche Salze organischer Basen, wie Mono-, Di- oder Triethanolamin, vorliegen. Vorzugsweise liegen die anionischen Tenside in Form ihrer Natrium- oder Kaliumsalze, insbesondere in Form der Natriumsalze, vor.

Als anionische Tenside werden erfindungsgemäß besonders bevorzugt Fettalkoholethersulfate, Alkylsulfate, Alkylbenzolsulfonate und/oder Alkansulfonate eingesetzt. Die anionischen Tenside sind vorzugsweise in einer Menge von 10 - 75, besonders bevorzugt 20 - 70, vor allem 30 - 70, insbesondere 40 - 65 Gew.%, in der Zusammensetzung enthalten.

Das erfindungsgemäße Mittel umfasst weiterhin Parfumstoffe (Duftstoffe). Neben den erfindungsgemäßen Bestandteilen kann das Sanitärmittel weitere übliche Bestandteile umfassen, beispielsweise Desinfektionsmittel, Konservierungsstoffe, wie zum Beispiels Isothiazolon-Derivate oder Schaumstabilisatoren, wie Kokosfettsäureamidopropylbetain oder Kokosfettsäureamidopropyldimethylaminoxid, Kokosfettsäuremono/diethanolamid oder Alkylpolyetherglycerol-ethersulfate, aber auch Farbstoffe und/oder Kalk- oder Urinstein lösende Substanzen, insbesondere Säuren.

Bei den Parfümstoffen handelt es sich um die aus dem Stand der Technik gängigen. Als Beispiele seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind zum Beispiel Benzylacetat, Phenoxyethylisobutyrat, p-ter.-Butylcyclohesyl-acetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylalicylat. Zu den Ethern zählen beispielsweise Benzylethylether, zu den Aldehyden zum Beispiel die linearen Alkanale mit 8 bis 18 Kohlenstoffatomen, Citral, Citronellal, Citronellyloxyacetaldehyd, Cylamenaldehyd, Hydroxycitronellal, Lilial und Bourgeonal, zu den Ketonen zum Beispiel die Jonone, α-Isomethylionen und Methylcedrylketon, zu den Terpenalkoholen Menthol, Anethol, Citronellol, Eugenol, Isoeugenol, Geraniol, Linalool, Nerol, Phenylethylalkohol, Tetrahydromyrcenol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen.

Auch ätherische Öle geringerer Flüchtigkeit, die meist als Aromakomponenten verwendet werden, eignen sich als Parfümöle, zum Beispiel Salbelöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandilöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller, Salbeiöl, β-Damascone, Geraniumöl Bourbon, Cyclohexylsalicylat, Vertofix Coeur, Iso-E-Super, Fixolide NP, Evemyl, Iraldein gamme, Phenylessigsäure, Geranylacetat, Benzylacetat, Rosenoxid, Romillat, Irotyl und Floramat allein oder in Mischungen, eingesetzt.

Die Menge der Dosierung ist abhängig von der gewünschten Duftintensität und liegt vorzugsweise im Bereich von 5 - 15, besonders bevorzugt von 8 - 12 Gew%.

Die verwendeten Parfumstoffe sollten gegenüber den Bleichmitteln in dem Gel im Wesentlichen beständig sein.

Auch ist es möglich, dem erfindungsgemäßen Mittel Olefinsulfonate, Ethersulfate und/oder Tenside, insbesondere Säuremethyltauride als Schäumer mit Selbstreinigungswirkung zuzusetzen.

Um das Austrocknen des Mittel zu verhindern, kann das Mittel zusätzlich noch vorzugsweise zwischen 5 und 15 Gew.%, aliphatische Di-, Oligo- oder Polyhydroxyverbindungen oder deren Ether oder der Verbindung(en) aus der Gruppe Glycerin, 1,3-Dihydroxypropan, 1,3- oder 1,4-Dihydroxybutan, 1,3-Dihydroxyisobutan und/oder Pentaerythrit umfassen.

Der Formulierung können - falls gewünscht - auch Salze, wie beispielsweise Natriumsulfat, hinzugegeben werden, um die Auflösegeschwindigkeit zu erhöhen. Der Salzanteil kann bis zu 10 Gew.%, vorzugsweise bis zu 5 Gew.%, betragen.

Das erfindungsgemäße Mittel kann in hygienischer Weise ohne Berührung von mit dem WC-Becken verbundenen, möglicherweise verunreinigten Vorrichtungen aufgebracht und erneuert werden.

Ein wesentlicher Vorteil des erfindungsgemäßen Mittels besteht darin, dass es nach den Wünschen des Verbrauchers portionierbar ist. Wünscht der Verbraucher eine intensivere Reinigung oder wird die Toilette häufig benutzt, so kann entsprechend stärker dosiert werden.

Das erfindungsgemäße Mittel kann auch auf einfache Weise gleichzeitig an verschiedenen Stellen des Sanitärgegenstands appliziert werden, beispielsweise, um sowohl auf der rechten, als auch auf der linken Seite einer Toilettenschüssel eine gleichmäßige Reinigungswirkung zu erzielen.

Die erreichte Haftung ist an dem Sanitärgegenstand selbst bei einer Anbringung an einer senkrechten Fläche so gut, dass sich das Mittel sogar unter der zusätzlichen Krafteinwirkung von Spülwasserströmen nicht ablöst.

Die erfindungsgemäßen Sanitärmittel sind erst nach einer größeren Anzahl von Spülvorgängen abspülbar. Die Anzahl der Spülvorgänge richtet sich natürlich nach der Zusammensetzung des jeweiligen Sanitärmittels, der aufgebrachten Menge sowie der Geometrie des aufgebrachten Sanitärmittels.

Vorzugsweise ist das erfindungsgemäße Mittel ein salbenartiges pastöses und/oder cremeartiges Gel. Die Gele sind im wesentlichen formstabil, so dass sie nicht "herunterlaufen" oder "tropfen".

Die Haftung und auch die Form der Gele bleibt trotz der durch die Wasserspülung einwirkenden erheblichen Kräfte (Reibung, Deformation ...) erhalten. Vorzugsweise umfassen diese pastösen Gele 1 bis 20 Gew.% Bleichmittel, etwa 1 Gew.% bis 25 Gew.%, insbesondere 5 bis 15 Gew.%, Duftstoffe, 1 Gew.% bis 20 Gew.%, insbesondere 5 bis 10 Gew.%. anionische Tenside, 20 Gew.% bis 40 Gew.% Haftvermittler, zwischen 1 und 20 Gew.% Feuchthaltemittel, 0 bis 1 Gew.% Verdicker und 0 Gew.% bis 5 Gew.%, vorzugsweise zwischen 0 und 1 Gew.%, Konservierungsmittel sowie etwa 35 bis 65 Gew.% Wasser.

In dieser bevorzugten Ausführungsform werden insbesondere α-Olefinsulfonate, Methyltauride und Ethersulfate als anionische Tenside verwendet.

Als anionisches Tensid, das gleichzeitig als Schäumer fungiert, hat sich insbesondere Alkyl(C12-C14)-polyethylenglykol-(2EO)-ethersulfattriisopropanol-ammoniumsalz bewährt. Dieses anionische Tensid ist bevorzugt, da es im Gegensatz zu Olefinsulfonat oder Taurid auch in höheren Konzentrationen nicht ausläuft.

Alkyl(C16-C18)-ethoxylat (30 EO) und Alkyl(C22)-ethoxylat (35 EO) werden bevorzugt als Haftvermittler und nichtionische Tenside eingesetzt. Das Mittel umfaßt weiterhin vorzugsweise 0 bis 3 Gew.% Aminoxide, wie zum Beispiel Kokosfettsäureamidopropyldimethylaminoxid (35 %ig), das von der Firma Rewo/Goldschmidt als Schaumstabilisator unter der Bezeichnung Rewominox B204 erhältlich ist und 0 bis 2 Gew.% Verdicker, wie beispielsweise einem Polysaccharid, insbesondere einem Xanthan-Gum, das zum Beispiel von Rhodia unter der Bezeichnung Rhodopol T erhältlich ist. Als Konservierungsmittel können beispielsweise Isothiazolon-Derivate verwendet werden.

Die Gele lassen sich vorzugsweise über Tuben, vergleichbar den Zahnpastatuben, Spritzen oder Kartuschen in das Toilettenbecken einbringen und bleiben dort für eine Vielzahl von aufeinanderfolgenden Spülvorgängen haften.

Die an einem Haake-Viskosimeter, System Platte-Kegel, Sensor PK 5 1°, Schergefälle von 25 s⁻¹ und 20°C zu bestimmenden Viskositäten dieser pastösen Gele sollten wenigstens 15.000 mPas, üblicherweise wenigstens 50.000 mPas, vorzugsweise wenigstens 90.000 mPas und besonders bevorzugt wenigstens 110.000 mPas, betragen.

Die Erfindung wird nachfolgend anhand eines Ausführungsbeispiels beschrieben.

### 1. Rezeptur des erfindungsgemäßen haftenden Sanitärmittels mit Bleichmittel Wasserstoffperoxid.

| Bestandteil | Gehalt (Gew. %) | |
|---|---|---|
| Alkl(C22)-ethoxylat (35EO) | 12,5 | Haftvermittler |
| Alkl(C16-C18)-ethoxylat (30EO) | 12,5 | Haftvermittler |
| Alkyl(C12-C14)-polyethylenglykol-(2EO)-ethersulfat-Triisopropanolammoniumsalz | 6 | anionisches Tensid |
| Glycerin (99%) | 10 | Feuchthaltemittel |
| Farbe | 0,02 | |
| Parfüm | 10 | |
| Wasserstoffperoxid (30%ig) | 2 | Bleichmittel |
| Wasser | ad 100 | |

Die Viskosität beträgt bei 20° C circa 120.000 mPas (Haake-Viskosimeter, System Platte-Kegel, Sensor PK 5 1°, Schergefälle 25 s⁻¹).

Als Alkyl(C22)-ethoxylat (35EO) wurde das Imbentin V001/99 der Firma Dr. W. Kolb AG eingesetzt, als Alkyl(C16-C18)-ethoxylat (30EO) das Imbentin 168S/300 der Firma Dr. W. Kolb GmbH AG, als Alkyl(C12-C14)-polyethylenglykol-(2EO)-ethersulfat-Triisopropanolammoniumsalz das Marlinat 242/90 T der Firma CONDEA GmbH und als Parfüm das Citrix Extra F548373 der Firma Quest International.

Das erfindungsgemäße Mittel wies bis zur vollständigen Auflösung eine glatte und transparente Oberfläche des Gels auf, auch bei einer niedrigen Spülfrequenz von vier Spülungen pro Tag. Der Auflösevorgang war weitgehend unabhängig von der Spülfrequenz.

### 2. Untersuchung der Lagerstabilisation des eingesetzten Wasserstoffperoxids

Es wurde ein Gel gemäß Rezeptur 1 hergestellt und acht Wochen bei Innentemperatur und Tageslicht eingelagert. Dann wurde hiervon eine 0,5 %ige wässrige Lösung hergestellt und mittels Teststreifen die H₂O₂-Konzentration bestimmt.

Als Nachweismethode wurde der Peroxid-Test "Merckoquant 1.10011" verwendet.

Es handelt sich hierbei um einen enzymatischen Test, bei dem Peroxidase (POD) den Peroxidsauerstoff auf einen organischen Redoxindikator überträgt und dadurch in ein blaues Oxidationsprodukt überführt.

Dabei konnte keine H₂O₂-Reduktion gegenüber einem frisch hergestellten Bleichmittel-Gel festgestellt werden.

Der Versuch belegt die Lagerstabilität des Bleichmittels in dem Mittel.

## Patentansprüche

1. Sanitärmittel zum Reinigen und Desinfizieren und zur Duftstoffabgabe, welches Mittel unmittelbar auf den Sanitärgegenstand applizierbar ist, dort haftet und erst nach einer größeren Anzahl von Spülvorgängen abspülbar ist und welches Mittel Lösungsmittel, insbesondere Wasser, Tenside und einen Haftvermittler, insbesondere aus der Gruppe der nichtionischen Tenside oder der nichtionischen Emulgatoren, ein Bleichmittel aus der Gruppe der sauerstofffreisetzenden Mittel, Duftstoffe sowie gegebenenfalls weitere übliche Bestandteile wie Verdicker, Farbstoffe, Konservierungsstoffe, Bleichmittel, Stabilisatoren und/oder Bleichmittelaktivatoren umfasst, wobei das Mittel ein formstabiles Gel und das Gel flüssigkristallin ist.

2. Sanitärmittel nach ein Anspruch 1, **dadurch gekennzeichnet, dass** die Viskosität des Mittels wenigstens 15.000 mPas, gemessen mit einem Haake-Viskosimeter, System Platte-Kegel, Sensor PK 5 1°, gemessen bei einem Schergefälle von 25 s⁻¹ und Raumtemperatur, beträgt.

3. Sanitärmittel nach einem der vorangegangenen Ansprüche, **dadurch gekennzeichnet, dass** das Bleichmittel aus der Gruppe der Carbonate Peroxyhydrate, Perborate, Persulfate, Peroxodisulfate, Peroxophosphate, Peroxonitrate, Caroate, Dithionite, Chlorate, Permanganate jeweils in der Form ihrer Alkali-, Ammonium- oder Erdalkalisalze, Wasserstoffperoxid und den organischen Peroxiden, ausgewählt wird, wobei unter den Alkalisalzen die Natrium- und Kaliumsalz und unter den Erdalkalisalzen die Calciumsalze besonders bevorzugt sind.

4. Sanitärmittel nach einem der vorangegangenen Ansprüche, **dadurch gekennzeichnet, dass** das Mittel zwischen 1 und 20 Gew.%, vorzugsweise zwischen 2 und 15 Gew.% und besonders bevorzugt zwischen 5 und 10 Gew.% Bleichmittel umfasst.

5. Sanitärmittel nach einem der vorangegangenen Ansprüche, **dadurch gekennzeichnet, dass** das Mittel zusätzlich ein Mittel zur Stabilisierung der Bleichmittel umfasst und das Stabilisierungsmittel aus der Gruppe der Harnstoffe, Barbitursäure, der hydrophoben Verbindungen und der Komplexbildner ausgewählt wird.

6. Sanitärmittel nach einem der vorangegangenen Ansprüche, **dadurch gekennzeichnet, dass** der Haftvermittler ein nichtionisches Tensid oder in nichtionischer Emulgator ist.

7. Sanitärmittel nach einem der vorangegangenen Ansprüche, **dadurch gekennzeichnet, dass** der Anteil der Duftstoffe in dem Mittel zwischen 5 und 15 Gew.% beträgt.

8. Verwendung eines Mittels nach einem der vorangegangenen Ansprüche als Toilettenreinigungs- und Beduftungsmittel.

## Claims

1. A sanitary agent for cleaning and disinfecting and for fragrance release, said agent being able to be applied directly to the sanitary object, adheres there and can be flushed away only after a relatively large number of flushing operations and which agent comprises solvents, in particular water, surfactants and an adhesion promoter, in particular from the group of nonionic surfactants or nonionic emulsifiers, a bleach from the group of oxygen-releasing agents, fragrances and optionally further customary constituents such as thickeners, dyes, preservatives, bleaches, stabilizers and/or bleach activators and wherein the sanitary agent is a dimensionally stable gel and the gel is liquid crystalline.

2. The sanitary agent as claimed in claim 1, **characterized in that** the viscosity of the agent is at least 15 000 mPas, measured using a Haake viscometer, plate-cone system, sensor PK 5 1°, measured at a shear gradient of 25 s⁻¹ and room temperature.

3. The sanitary agent as claimed in claim 1 or 2, **characterized in that** the bleach is selected from the group of carbonate, peroxyhydrates, perborates, persulfates, peroxodisulfates, peroxophosphates, peroxonitrates, caroates, dithionites, chlorates, permanganates, in each case in the form of their alkali metal, ammonium or alkaline earth metal salts, the hydrogen peroxide and the organic peroxides, where among the alkali metal salts, the sodium and potassium salts are particularly preferred, and among the alkaline earth metal salts, the calcium salts are particularly preferred.

4. The sanitary agent as claimed in one of the preceding claims, **characterized in that** the agent comprises between 1 and 20% by weight, preferably between 2 and 15% by weight and particularly preferably between 5 and 10% by weight of bleaches.

5. The sanitary agent as claimed in one of the preceding claims, **characterized in that** the agent additionally comprises an agent for stabilizing the bleaches and the stabilizing agent is selected from the group of ureas, barbituric acid, hydrophobic compounds and complexing agents.

6. The sanitary agent as claimed in one of the preceding claims, **characterized in that** the adhesion promoter is a non-ionic surfactant or a non-ionic emulsifier.

7. The sanitary agent as claimed in one of the preceding claims, **characterized in that** the agent comprises between 5 and 15 % of weight of fragrances.

8. The use of the sanitary agent as claimed in one of the preceding claims for toilet cleaning and fragrance release.

## Revendications

1. Agent sanitaire pour le nettoyage et la désinfection et pour la libération d'un parfum, l'agent pouvant être appliqué directement sur l'objet sanitaire, y adhérant et ne pouvant être éliminé par rinçage qu'après un nombre important de cycles de rinçage et ledit agent contenant des solvants, en particulier l'eau, des agents tensioactifs et un promoteur d'adhérence, en particulier du groupe des agents tensioactifs non ioniques ou des émulsifiants non ioniques, un agent de blanchiment du groupe des agents libérant de l'oxygène, des parfums ainsi que le cas échéant d'autres constituants usuels tels que des épaississants, des colorants, des conservateurs, des agents de blanchiment, des stabilisateurs et/ou des activateurs des agents de blanchiment, où l'agent est un gel de forme stable et le gel est de type à cristaux liquides.

2. Agent sanitaire selon la revendication 1, **caractérisé en ce que** la viscosité de l'agent est d'au moins 15 000 mPa.s, mesurée avec un viscosimètre de Haake, système plaque-cône, sonde PK 5 1°, mesurée à un gradient de cisaillement de 25 s⁻¹ et à température ambiante.

3. Agent sanitaire selon quelconque l'une des revendications précédentes, **caractérisé en ce que** l'agent de blanchiment est choisi dans le groupe formé par les carbonates peroxyhydratés, les perborates, les persulfates, les peroxodisulfates, les peroxophosphates, les peroxonitrates, les caroates, les dithionites, les chlorates, les permanganates, à chaque fois sous forme de leurs sels de métal alcalin, d'ammonium ou de métal alcalino-terreux, le peroxyde d'hydrogène et les peroxydes organiques, en préférant en particulier parmi les sels de métal alcalin les sels de sodium et de potassium et parmi les sels de métal alcalino-terreux les sels de calcium.

4. Agent sanitaire selon quelconque l'une des revendications précédentes, **caractérisé en ce que** l'agent comprend entre 1 et 20% en poids, de préférence entre 2 et 15% en poids et de manière particulièrement préférée entre 5 et 10% en poids d'agent de blanchiment.

5. Agent sanitaire selon quelconque l'une des revendications précédentes, **caractérisé en ce que** l'agent comprend en outre un agent pour la stabilisation des agents de blanchiment et **en ce que** l'agent de stabilisation est choisi dans le groupe formé par les urées, l'acide barbiturique, les composés hydrophobes et les complexants.

6. Agent sanitaire selon quelconque l'une des revendications précédentes, **caractérisé en ce que** le promoteur d'adhérence est un agent tensioactif non ionique ou un émulsifiant non ionique.

7. Agent sanitaire selon quelconque l'une des revendications précédentes, **caractérisé en ce que** la proportion de parfum dans l'agent se situe entre 5 et 15% en poids.

8. Utilisation d'un agent selon l'une quelconque des revendications précédentes comme agent de nettoyage des toilettes et parfum.
